# EUROPEAN PATENT APPLICATION

(11) **EP 2 491 810 A1**
(43) Date of publication of application: **29.08.2012**
(21) Application number: 12152396.3
(22) Date of filing: 25.01.2012
(51) Int. Cl.: A45C 13/02, A61B 5/022, A61B 5/021, A61B 19/02

(54) **Sphygmomanometer accommodation case and blood pressure measurement device including the same**

(30) Priority: 28.02.2011 JP 2011043043
(71) Applicant: Panasonic Corporation, Kadoma-shi Osaka 571-8501 (JP)
(72) Inventor: Fumuro, Shinichi, Chuo-ku, Osaka 540-6207 (JP); Nishibori, Yuichi, Chuo-ku, Osaka 540-6207 (JP)
(74) Representative: Appelt, Christian W.

(57) **Abstract**

A sphygmomanometer accommodation case (10) includes a case body (20) that accommodates an electronic sphygmomanometer (40) including a sphygmomanometer body (50), which is provided with an operation unit (51), and a cuff (60), which is separated from the sphygmomanometer body (50). A partition member (31) is arranged between the sphygmomanometer body (50) and the cuff (60) in the case body (20). The partition member (31) includes an opening (32) arranged at a portion corresponding to the operation unit (51).

## Description

This application is based upon and claims the benefit of priority from prior Japanese Patent Application No. 2011-043043, filed on February 28, 2011, the entire contents of which are incorporated herein by reference.

The present invention relates to a sphygmomanometer accommodation case that accommodates an electronic sphygmomanometer, which includes a sphygmomanometer body provided with an operation unit and a cuff discrete from the sphygmomanometer body, and a blood pressure measurement device that includes the case and the electronic sphygmomanometer.

Japanese Laid-Open Patent Publication No. 2010-99384 describes a prior art example of an electronic sphygmomanometer. The electronic sphygmomanometer includes a sphygmomanometer body, which is provided with an operation unit, and a cuff, which is connected by a tube to the sphygmomanometer body and which constricts the arm.

When the sphygmomanometer body and the cuff are accommodated in an accommodation case, the operation unit of the sphygmomanometer body may be pushed by the cuff. This may result in unnecessary power consumption of the electronic sphygmomanometer.

It is an object of the present invention to provide a sphygmomanometer accommodation case having a structure that avoids a situation in which the operation unit of the sphygmomanometer body is pushed by the cuff in the sphygmomanometer accommodation case.

One aspect of the present invention is a sphygmomanometer accommodation case including a case body that accommodates an electronic sphygmomanometer including a sphygmomanometer body, which is provided with an operation unit, and a cuff, which is separated from the sphygmomanometer body. A partition member is arranged between the sphygmomanometer body and the cuff in the case body. The partition member includes an opening arranged at a portion corresponding to the operation unit.

In the above aspect of the present invention, the opening is a hole formed in the partition member.

In the above aspect of the present invention, the case body includes a rectangular cuboidal-shaped container including an accommodation compartment, the partition member is plate-like and includes a lower surface facing a bottom wall of the container and inclined with respect to the bottom wall, and the partition member includes a lower edge adjacent to the bottom wall of the container and an upper edge adjacent to an open top of the container.

In the above aspect of the present invention, the lower edge is located in a front part of the container, and the upper edge is located at a rear part of the container.

In the above aspect of the present invention, a clearance is formed between the upper edge of the partition member and one inner surface of the container.

In the above aspect of the present invention, the clearance has a smaller width than the cuff.

In the above aspect of the present invention, the upper edge of the partition member includes two ends, each attached to the container by an elastic joining member.

In the above aspect of the present invention, the operation unit is projected from a surface of the sphygmomanometer body by a projected amount, and the opening has a depth that is greater than the projected amount of the operation unit.

In the above aspect of the present invention, the partition member includes a cuff support surface that supports the cuff, and the cuff support surface is defined on a rigid member having a higher rigidity than the partition member.

In the above aspect of the present invention, the sphygmomanometer body includes a display, and the partition member includes a transparent portion corresponding to at least the display.

In the above aspect of the present invention, the sphygmomanometer body includes a display, and the partition member covers the entire display.

In the above aspect of the present invention, the partition member partitions an interior of the case body into lower and upper compartments that accommodate the sphygmomanometer body and the cuff, respectively.

In the above aspect of the present invention, the operation unit includes a power button, and the power button automatically enters the opening of the partition member when the sphygmomanometer body is accommodated in the case body.

A further aspect of the present invention is a blood pressure measurement device including the sphygmomanometer accommodation case according to the above aspect and the electronic sphygmomanometer accommodated in the accommodation case.

In the further aspect of the present invention, the case body includes a rectangular cuboidal-shaped container including an accommodation compartment. The partition member is a plate-like member including a lower surface facing a bottom wall of the container and inclined with respect to the bottom wall. The partition member includes a lower edge adjacent to the bottom wall of the container and an upper edge adjacent to an open top of the container, the lower edge is located in a front part of the container, and the upper edge is located in a rear part of the container, the electronic sphygmomanometer includes a bottom surface, a front surface inclined with respect to the bottom surface, and a display arranged on the inclined front surface, and the partition member partitions the accommodation compartment into an upper compartment, which accommodates the cuff, and a lower compartment, which accommodates the sphygmomanometer body.

The present invention provides a sphygmomanometer accommodation case having a structure that avoids a situation in which the operation unit of the sphygmomanometer body is pushed by the cuff in the sphygmomanometer accommodation case.

Other aspects and advantages of the present invention will become apparent from the following description, taken in conjunction with the accompanying drawings, illustrating by way of example the principles of the invention.

The invention, together with objects and advantages thereof, may best be understood by reference to the following description of the presently preferred embodiments together with the accompanying drawings in which:
Fig. 1 is a perspective view showing a blood pressure measurement device according to one embodiment of the present invention;
Fig. 2(a) is a front view showing a protector of a sphygmomanometer accommodation case of Fig. 1;
Fig. 2(b) is a cross-sectional view of the protector taken along line B-B in Fig. 2(a);
Fig. 3 is a cross-sectional view of the blood pressure measurement device taken along line A-A in Fig. 1;
Fig. 4 is a perspective view showing the blood pressure measurement device in a state in which an electronic sphygmomanometer is accommodated in the blood pressure accommodation case;
Fig. 5 is a cross-sectional view of the blood pressure measurement device of Fig. 4;
Fig. 6 is a cross-sectional view showing how to accommodate the electronic sphygmomanometer in the accommodation case; and
Figs. 7 to 9 are cross-sectional views showing modifications of a blood pressure measurement device according to the present invention in a state in which the electronic sphygmomanometer is accommodated in the accommodation case.

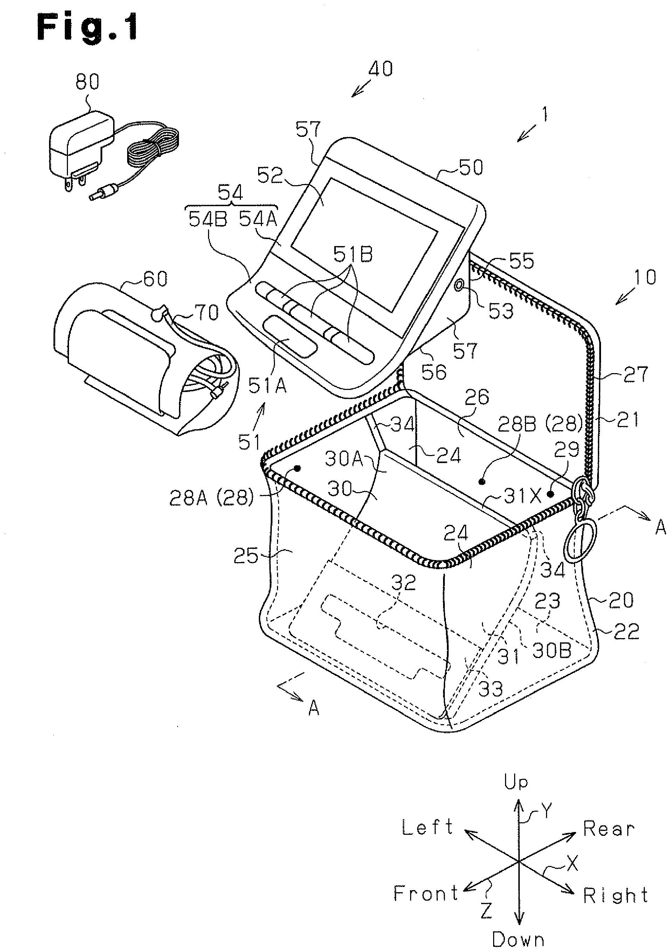

A blood pressure measurement device 1 according to one embodiment will now be described with reference to Fig. 1.

In the description hereafter, the upward, downward, leftward, rightward, frontward, and rearward directions are as indicated by the orthogonal coordinate axes in each drawing.

The blood pressure measurement device 1 includes an electronic sphygmomanometer 40 and an accommodation case 10, which accommodates the electronic sphygmomanometer 40. The electronic sphygmomanometer 40 is accommodated in the accommodation case 10 when the blood pressure measurement device 1 is not used, such as when storing or carrying the blood pressure measurement device 1.

The electronic sphygmomanometer 40 includes a sphygmomanometer body 50, a cuff 60, and a power supply adapter 80. The sphygmomanometer body 50 includes a processing unit that controls blood pressure measurement operations and calculates measurement result. The cuff 60 is wrapped around the arm of a user to constrict the arm. The power supply adapter 80 supplies power from a commercial AC power supply to the sphygmomanometer body 50. The cuff 60 is connected by a tube 70 to the sphygmomanometer body 50.

The sphygmomanometer body 50 includes an operation unit 51 operated by the user for blood pressure measurement or the like, a display 52 that displays measurement results or the like, and a tube connector 53 for connection with the tube 70. Further, the sphygmomanometer body 50 incorporates an air pump and a battery (both not shown). The air pump supplies air through the tube 70 to an air bag of the cuff 60. The battery serves as a power supply. The display 52 is, for example, a liquid crystal display panel.

The operation unit 51 includes push-type buttons, namely, a power button 51A and measurement buttons 51 B. The buttons 51 A and 51B each project outward from an operation surface 54B of the sphygmomanometer body 50. The user's tactile feel of the buttons 51 A and 51B is taken into account to set the projected amount P of the buttons 51 A and 51 B from the operation surface 54B.

The sphygmomanometer body 50 has five outer surfaces, namely, a front surface 54, a rear surface 55, a bottom surface 56, and right and left surfaces 57. A display surface 54A of the display 52 and the operation surface 54B of the operation unit 51 are defined in the front surface 54.

The display surface 54A and the operation surface 54B are inclined with respect to the bottom surface 56. In the illustrated example, an inclination angle of the display surface 54A with respect to the bottom surface 56 is greater than an inclination angle of the operation surface 54B with respect to the bottom surface 56.

The accommodation case 10 includes a case body 20 and a protector 30. The case body 20 includes an accommodation compartment 28 that is adapted to accommodate the electronic sphygmomanometer 40. The protector 30 prevents contact of the cuff 60, tube 70, and power supply adapter 80 with the sphygmomanometer body 50 in the accommodation compartment 28.

The case body 20 includes a rectangular cuboidal-shaped container 22 having an open top, a lid 21 connected to the container 22 to close the open top of the container 22, and a fastener such as a line fastener 27 that holds the lid 21 in a state closing the open top of the container 22. The line fastener 27 includes elements attached to the rim of the open top of the container 22 and the rim of the lid 21.

The container 22 includes five walls that partition the accommodation compartment 28, namely, a bottom wall 23, right and left walls 24, a front wall 25, and a rear wall 26. The bottom wall 23, the two side walls 24, the front wall 25, and the rear wall 26 are also referred to as the inner surfaces of the container 22. In one embodiment, the accommodation compartment 28 may be a generally rectangular cuboidal accommodation compartment.

The protector 30 partitions the accommodation compartment 28 into two, namely, an upper compartment 28A and a lower compartment 28B. The upper compartment 28A is adapted to accommodate the cuff 60, the tube 70, and the power supply adapter 80. When the lid 21 is closed, the upper compartment 28A is surrounded by an upper surface 30A of the protector 30, the lid 21, the two side walls 24, and the front wall 25. The lower compartment 28B is adapted to accommodate the sphygmomanometer body 50. The lower compartment 28B is surrounded by a lower surface 30B of the protector 30, the bottom wall 23, the two side walls 24, and the rear wall 26.

A gap, or clearance 29, is formed between the rear wall 26 of the container 22 and an upper edge 31X of the protector 30 so that the sphygmomanometer body 50 can be arranged in the lower compartment 28B . The clearance 29 extends throughout the entire length between the side walls 24 of the container 22.

The operation of the electronic sphygmomanometer 40 will now be described.

When using the electronic sphygmomanometer 40, the cuff 60 is connected to the sphygmomanometer body 50 by the tube 70. When a commercial AC power supply is available, the power supply adapter 80 connects to the commercial AC power supply to operate the sphygmomanometer body 50. When the power supply adapter 80 is not connected to the sphygmomanometer body 50, the incorporated battery is used as the power supply of the sphygmomanometer body 50.

When measuring blood pressure, the user pushes the power button 51 A of the operation unit 51 to activate the sphygmomanometer body 50. Then, the cuff 60 is set on an arm, and the electronic sphygmomanometer 40 starts to measure blood pressure when the measurement button 51 B is pushed. The processing unit of the sphygmomanometer body 50 performs data processing in accordance with the operation of the measurement buttons 51 B, such as storage of the present measurement result, display of the past measurement results, and deletion of past measurement results.

The air pump supplies air to the cuff 60 through the tube 70 in response to the pushing of the measurement buttons 51 B. The cuff 60 constricts the user's arm.

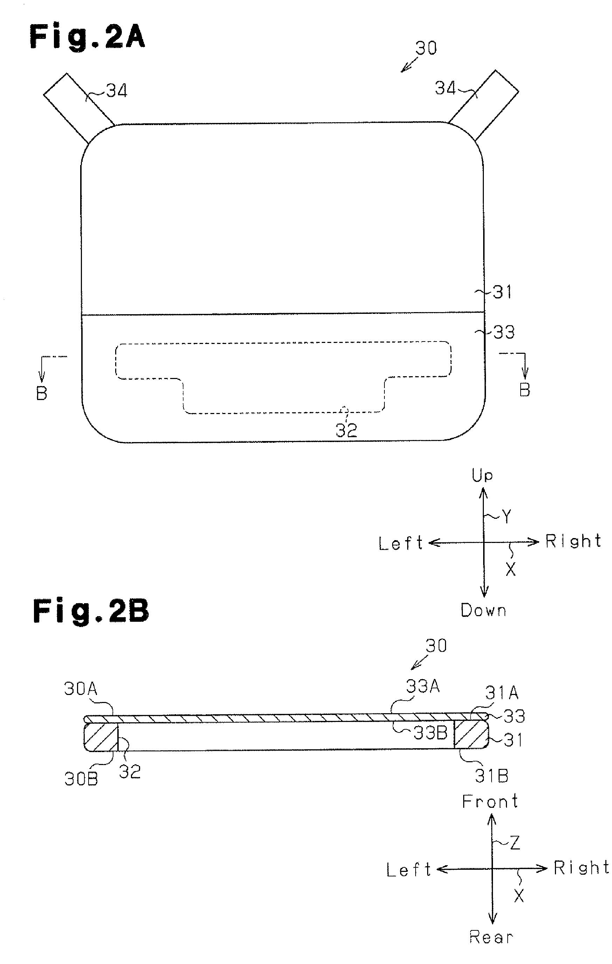

The protector 30 will now be described in detail with reference to Figs. 2A and 2B.

The protector 30 includes a plate-like rectangular partition member 31, a plate-like rigid member 33 having a higher rigidity than the partition member 31, and joining members 34 that join the partition member 31 with the container 22. The partition member 31 can be formed from a flexible foam material. The rigid member 33 can be formed from polyethylene terephthalate (PET). The joining members 34 can be formed from an elastic member, such as a rubber band having high elasticity.

The rigid member 33 is attached to the lower part of an upper surface 31A of the partition member 31. In other words, a lower surface 33B of the rigid member 33 is fixed to the upper surface 31A of the partition member 31.

The partition member 31 includes an opening 32, which is a hole, arranged in a portion corresponding to the operation unit 51 of the sphygmomanometer body 50. The operation unit 51 of the sphygmomanometer body 50 fits into the opening 32. The upper surface 31A and lower surface 31 B of the partition member 31 have a larger area than the front surface 54 of the sphygmomanometer body 50.

The upper surface 33A of the rigid member 33 and the upper surface 31A of the partition member 31 define the upper surface 30A of the protector 30. The lower surface 31 B of the partition member 31 defines the lower surface 30B of the protector 30. The upper surface 30A serves as a cuff support surface.

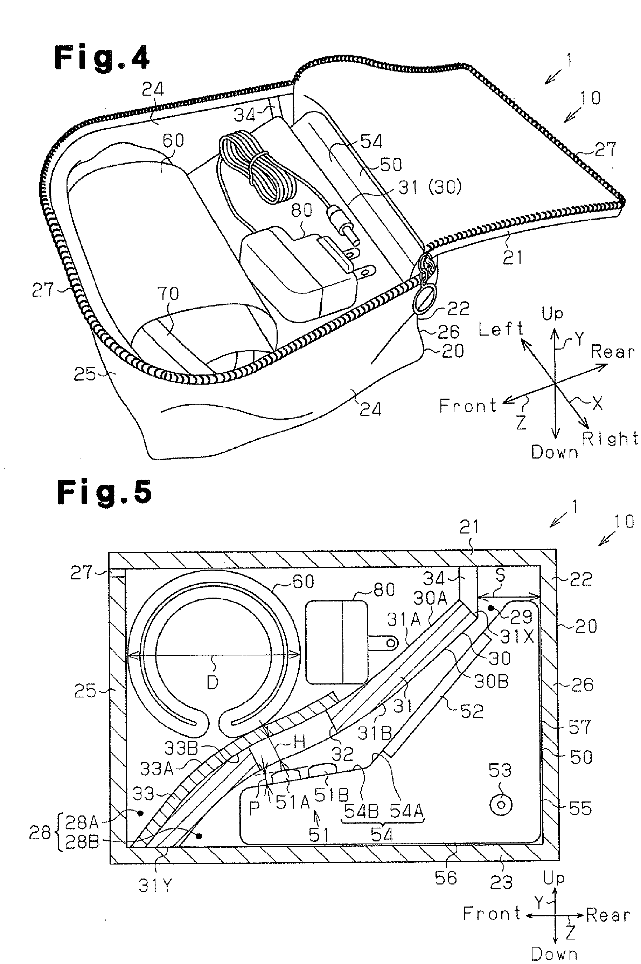

As shown in Fig. 5, the opening 32 has a depth H that is greater than the projected amount P of the power button 51 A and measurement buttons 51 B of the sphygmomanometer body 50.

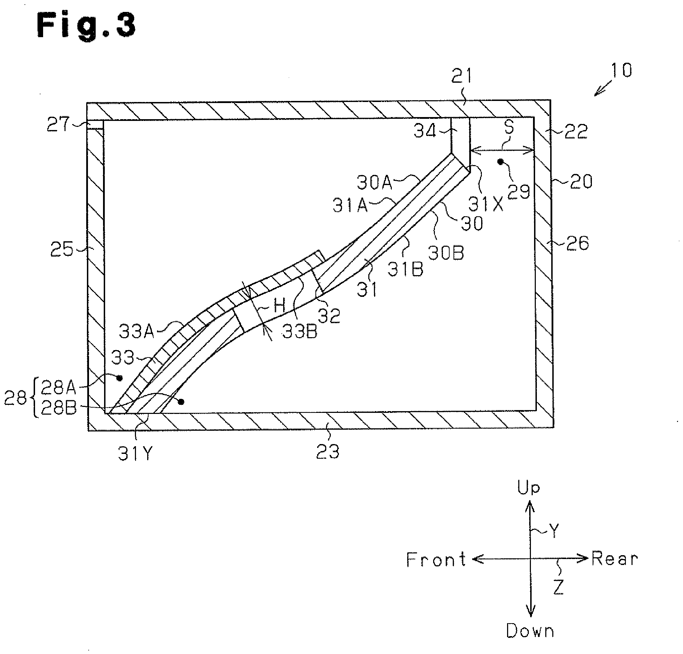

The accommodation case 10 will now be described in detail with reference to Fig. 3.

The partition member 31 includes a lower edge 31Y, which is adjacent to the bottom wall 23 of the container 22, and an upper edge 31X, which is adjacent to the open top of the container 22. Further, the lower edge 31Y of the partition member 31 is adjacent to the front wall 25 of the container 22, and the upper edge 31X of the partition member 31 is adjacent to the rear wall 26 of the container 22. Accordingly, the partition member 31 is inclined in the container 22. In the illustrated example, the partition member 31 is attached to the container 22 so that the upper surface 31A of the partition member 31 faces a diagonally frontward direction.

The upper surface 31A of the partition member 31 faces the lid 21 and the front wall 25 of the container 22 when the lid 21 is closed. The lower surface 31 B of the partition member 31 faces the bottom wall 23 of the container 22 and the rear wall 26 of the container 22.

The lower edge 31Y of the partition member 31 is fixed to the bottom wall 23 of the container 22 and arranged parallel to and near the front wall 25 of the container 22. The upper edge 31X of the partition member 31 is arranged parallel to and near the rear wall 26 of the container 22. However, the upper edge 31X is separated from the rear wall 26.

The left end of the upper edge 31X of the partition member 31 is joined with the left wall 24 by one of the joining members 34. The right end of the upper edge 31X of the partition member 31 is joined with the right wall 24 with the other joining member 34 (refer to Fig. 1).

A state in which the electronic sphygmomanometer 40 is accommodated in the accommodation case 10 will now be described with reference to Figs. 4 and 5. In Fig. 5, the tube 70 is not shown, and the structure of the electronic sphygmomanometer 40 is simplified.

As shown in Fig. 4, the electronic sphygmomanometer 40 is accommodated in the accommodation case 10 with the cuff 60 and the power supply adapter 80 removed from the sphygmomanometer body 50. The tube 70 is connected to the cuff 60.

The sphygmomanometer body 50 is arranged at the lower side of the protector 30. The cuff 60 and the power supply adapter 80 are arranged at the upper side of the protector 30. The cuff 60 is arranged at a location near the front wall 25 of the accommodation case 10, and the power supply adapter 80 is arranged rearward from the cuff 60.

As shown in Fig. 5, when the sphygmomanometer body 50 is arranged in the lower compartment 28B, the rear surface 55 of the sphygmomanometer body 50 faces the rear wall 26 of the container 22, the bottom surface 56 of the sphygmomanometer body 50 faces the bottom wall 23 of the container 22, and the front surface 54 of the sphygmomanometer body 50 faces the lower surface 31 B of the partition member 31. The front surface 54 of the sphygmomanometer body 50 is entirely covered by the lower surface 31 B of the partition member 31. Preferably, the front surface 54 is spaced apart from the lower surface 31B of the partition member 31. The upper end of the sphygmomanometer body 50 projects through the clearance 29 into the upper compartment 28A.

The cuff 60 is supported by the rigid member 33. The front part, upper part, and lower part of the cuff 60 are respectively in contact with the front wall 25 of the container 22, the lid 21, and the upper surface 33A of the rigid member 33.

The distance between the upper edge 31X of the partition member 31 and the rear wall 26 of the container 22 in the front to rear direction Z is the opening width S of the clearance 29. The diameter of the cuff 60 in the accommodated state is referred to as the cuff width D. The opening width S is smaller than the cuff width D when force is not applied to the partition member 31.

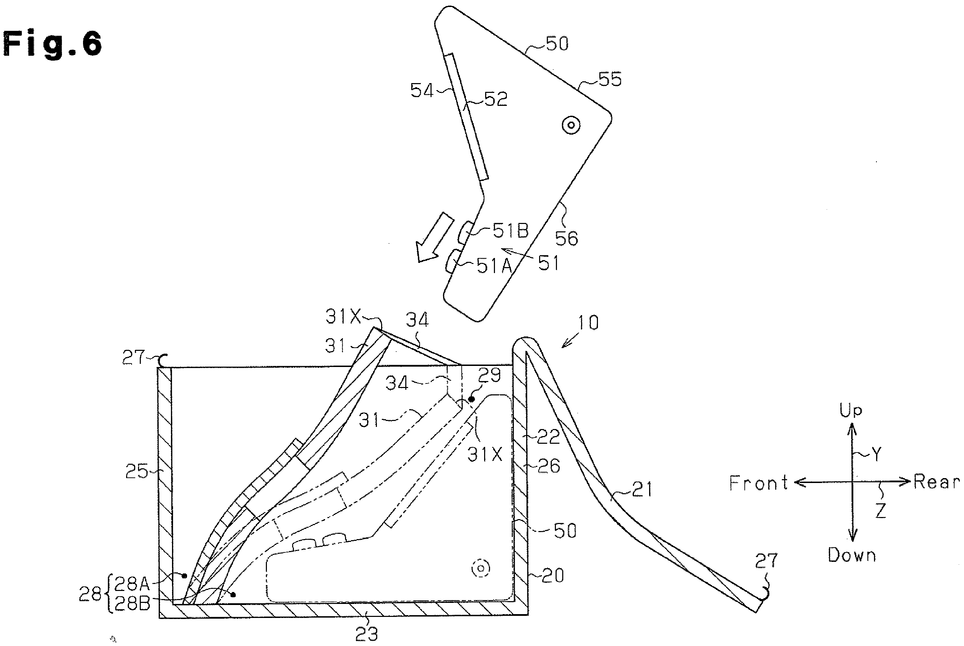

The procedures for accommodating the electronic sphygmomanometer 40 in the accommodation case 10 will now be described with reference to Fig. 6. In Fig. 6, the sphygmomanometer body 50 shown by solid lines indicates the state of procedure 2, and the sphygmomanometer body 50 shown by broken lines indicates the state of procedure 3.
Procedure 1: opening the line fastener 27.
Procedure 2: lifting the upper edge 31X of the partition member 31 from the position shown by broken lines to the position shown by solid lines.
Procedure 3: inserting the sphygmomanometer body 50 into the lower compartment 28B through the clearance 29.
Procedure 4: returning the upper edge 31X of the partition member 31 from the position shown by solid lines to the position shown by broken lines.
Procedure 5: arranging the cuff 60 above the protector 30 in the upper compartment 28A.
Procedure 6: arranging the power supply adapter 80 above the protector 30 in the upper compartment 28A.
Procedure 7: engaging the elements of the line fastener 27 with each other.

The blood pressure measurement device of the present embodiment has the advantages described below.
(1) The blood pressure measurement device 1 includes the partition member 31, which is arranged between the sphygmomanometer body 50 and the cuff 60 and includes the opening 32 formed at a portion corresponding to the operation unit 51 of the sphygmomanometer body 50. This structure prevents the operation unit 51 of the sphygmomanometer body 50 from being pushed by the cuff 60 or the power supply adapter 80 when the sphygmomanometer body 50, the cuff 60, and the power supply adapter 80 are accommodated in the sphygmomanometer accommodation case 10.
(2) The opening 32 is a hole formed in the partition member 31. This structure reduces the trouble for forming the opening 32 compared to when forming a recess in the partition member 31 in lieu of the hole that serves as the opening 32.
(3) The partition member 31 is inclined in the container 22 with the lower edge 31Y of the partition member 31 adjacent to the bottom wall 23 of the container 22, and the upper edge 31X of the partition member 31 adjacent to the open top of the container 22. The front surface 54 of the sphygmomanometer body 50 is inclined with respect to the bottom surface 56 of the sphygmomanometer body 50. The sphygmomanometer body 50 is accommodated in the lower compartment 28B of the accommodation case 10. The cuff 60 is accommodated in the upper compartment 28A of the accommodation case 10. This structure allows for reduction in size of the accommodation case 10 compared to a structure that accommodates the sphygmomanometer body 50 and the cuff 60 next to each other in the front to rear direction Z of the accommodation case 10.
(4) The partition member 31 is attached to the container 22 so that the upper surface 31 A of the partition member 31 faces a diagonally forward direction. In this structure, the sphygmomanometer body 50, which is the largest one of the various elements of the electronic sphygmomanometer 40, is arranged faced frontward in the lower compartment 28B, and the other elements of the electronic sphygmomanometer 40 is arranged in the upper compartment 28A. When the user looks at container 22 from the front surface, the user can easily recognize whether or not the various elements of the electronic sphygmomanometer 40 are accommodated in the accommodation compartment 28.
(5) The clearance 29 is formed between the upper edge 31X of the partition member 31 and the rear wall 26, which is one inner surface of the container 22. In this structure, when accommodating the sphygmomanometer body 50 in the lower compartment 28B, the user can lift the partition member 31 from the clearance 29. Thus, the sphygmomanometer body 50 can easily be arranged in the lower compartment 28B compared to a structure that does not have the clearance 29.
(6) The upper edge of the sphygmomanometer body 50 arranged in the lower compartment 28B projects upward from the clearance 29. This structure allows for easy visual recognition of whether the sphygmomanometer body 50 is accommodated in the lower compartment 28B when the lid 21 of the accommodation case 10 is open.
(7) The opening width S of the clearance 29 is smaller than the cuff width D of the cuff 60.
   This structure makes it difficult to erroneously arrange the cuff 60 in the lower compartment 28B. In the present embodiment, the user needs to forcibly lift the upper edge 31X of the partition member 31 to enlarge the opening width S of the clearance 29 when arranging the cuff 60 in the lower compartment 28B. In contrast, in a comparative example in which the opening width S of the clearance 29 is greater than the cuff width D of the cuff 60, the user can easily place the cuff 60 in the lower compartment 28B without lifting the upper edge 31X of the partition member 31. Accordingly, the embodiment avoids a situation in which the user erroneously arranges the cuff 60 in the lower compartment 28B.
(8) The two ends of the upper edge 31X of the partition member 31 are attached to the container 22 by the joining members 34, which are elastic. In this structure, the user can easily change the opening width S of the clearance 29 by moving the upper edge 31X of the partition member 31. When the user releases the force applied to move the partition member 31, the partition member 31 automatically returns to its initial position due to the resilient force of the joining members 34, and the opening width S of the clearance 29 automatically returns its the initial value.
(9) The operation unit 51 (buttons 51A and 51B) is projected by the projected amount P from the front surface 54 of the sphygmomanometer body 50. The opening 32 of the partition member 31 in the accommodation case 10 has a depth H that is greater than the projected amount P of the operation unit 51 (buttons 51A and 51 B). In this structure, when the sphygmomanometer body 50 is arranged in the lower compartment 28B, the distal surfaces of the buttons 51A and 51B in the operation unit 51 are located in the opening 32. This prevents the operation unit 51 from being pushed by contact with the rigid member 33.
(10) The opening 32 of the partition member 31 is covered by the rigid member 33. In this structure, the cuff 60 arranged in the upper compartment 28A is prevented from contacting the operation unit 51 through the opening 32. This avoids a situation in which the power button 51A and the measurement buttons 51B are pushed by the cuff 60.
(11) The partition member 31 covers the entire display 52 of the sphygmomanometer body 50. In this structure, the power supply adapter 80 arranged in the upper compartment 28A is prevented from contacting the display 52 of the sphygmomanometer body 50.
(12) In a comparative example in which the lower edge 31Y is not fixed to the bottom wall 23, the user may erroneously arrange the cuff 60 in the lower compartment 28B. In contrast, in the present the embodiment, the lower edge 31Y of the partition member 31 is fixed to the bottom wall 23 of the container 22. Thus, there is no opening between the lower edge 31 Y and the bottom wall 23 through which the cuff 60 can be inserted. This avoids a situation in which the erroneously arranges the cuff 60 in the lower compartment 28B.

It should be apparent to those skilled in the art that the present invention may be embodied in many other specific forms without departing from the scope of the invention. Particularly, it should be understood that the present invention may be embodied in the following forms.

In the embodiment described above, the opening 32 of the partition member 31 in correspondence with the power button 51A and the measurement buttons 51B. However, the size and shape of the opening 32 may be changed as described below.
(A) The size and shape of the opening 32 may be changed to correspond to only the power button 51A. In such a structure, the power button 51A corresponds to the operation unit.
(B) The size of the opening 32 may be changed to a size greater than the size corresponding to the power button 51A and the measurement buttons 51 B.

In the embodiment described above, a hole is formed as the opening 32 in the partition member 31. However, a recess may be formed in part of the partition member 31 as the opening 32 in lieu of the hole. The recess is depressed toward the upper surface 31A. The recess has a greater recess than the projected amount P of the buttons 51A and 51 B.

In the above modification, the depth of the recess serving as the opening 32 may be less than the projected amount P of the buttons 51 A and 51 B.

In the embodiment described above, the opening width S of the clearance 29 is set to be smaller than the cuff width D of the cuff 60. However, the opening width S of the clearance 29 may be greater than the cuff width D of the cuff 60.

In the embodiment described above, the protector 30 and the container 22 form the clearance 29 when force is not applied to the partition member 31. However, the structure related with the clearance 29 may be modified in the following manner. The protector 30 and the container 22 may be formed so that the clearance 29 is closed when force is not applied to the partition member 31. In such a state, the opening width S of the clearance 29 is zero. In this structure, the clearance 29 is formed between the partition member 31 and the container 22 when the user lifts the upper edge 31X of the partition member 31.

In the embodiment described above, the partition member 31 covers the entire front surface 54 of the sphygmomanometer body 50. However, the partition member 31 may cover only the operation surface 54B.

In the embodiment described above, the protector 30 includes the rigid member 33. However, the rigid member 33 may be eliminated from the protector 30.

In the embodiment described above, the rigid member 33 is formed from polyethylene terephthalate (PET). However, the rigid member 33 may be formed from a flexible foam material.

In the embodiment described above, the rigid member 33 covers only the lower part of the partition member 31. However, the rigid member 33 may cover the entire partition member 31.

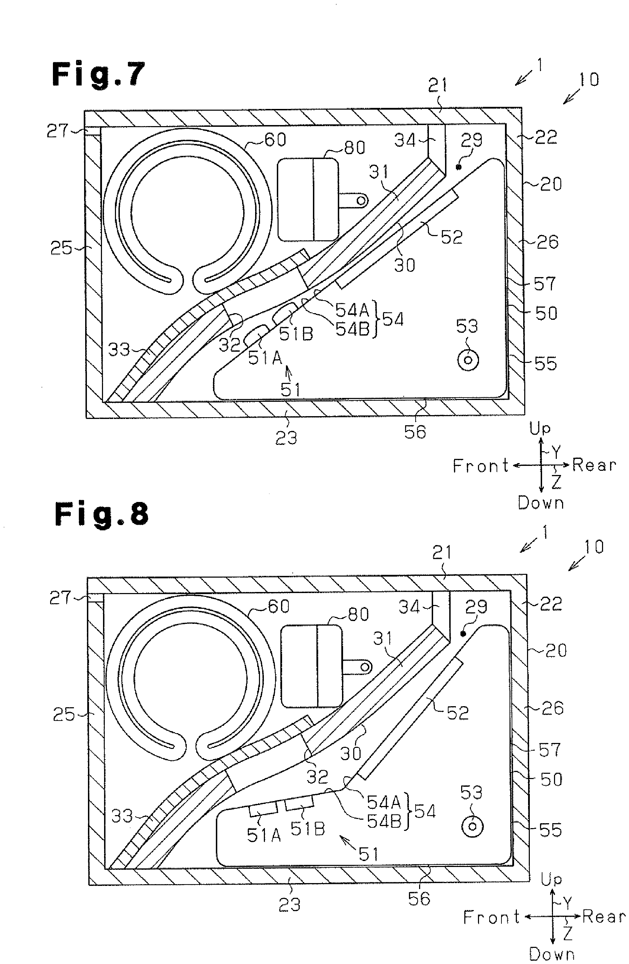

In the embodiment described above, the display surface 54A and the operation surface 548 are set at different inclination angles with respect to the bottom surface 56 in the sphygmomanometer body 50. However, the display surface 54A and the operation surface 54B may be set at the same inclination angle with respect to the bottom surface 56, as shown in Fig. 7.

In the embodiment described above, the operation unit 51 is arranged below the display 52 in the front surface 54 of the sphygmomanometer body 50. However, the operation unit 51 may be arranged leftward, rightward, or upward from the display 52.

In the embodiment described above, the power button 51A and the measurement button 51 B are projected from the operation surface 54B in the sphygmomanometer body 50. However, the sphygmomanometer body 50 may be formed so that the buttons 51 A and 51 B are not projected from the operation surface 54B, as shown in Fig. 8.

In such a structure, the opening 32 is formed at the portion corresponding to the buttons 51A and 51B of the partition member 31. This avoids a situation in which the buttons 51 A and 51B are pushed by contact with the partition member 31. Further, an advantage similar to advantage (9) is obtained even when the depth H of the opening 32 is less than the projected amount P of the above embodiment. In other words, an advantage similar to advantage (9) of the above embodiment is obtained even if the thickness of the partition member 31 less than that of the above embodiment.

In the blood pressure measurement device 1, the entire partition member 31 is formed from an opaque material. However, the portion of the partition member 31 corresponding to the display 52 of the sphygmomanometer body 50 may be formed from a transparent member. In such a case, the portion corresponding to the display 52 may be formed from a transparent material, and the surrounding portion may be formed from an opaque material. Alternatively, the partition member 31 may entirely be formed from a transparent material.

In such a structure, the display 52 of the sphygmomanometer body 50 can be visually recognized through the transparent portion of the partition member 31 in a state in which the sphygmomanometer body 50 is arranged in the lower compartment 28B. Thus, the user can easily check whether or not the sphygmomanometer body 50 arranged in the lower compartment 28B is operating.

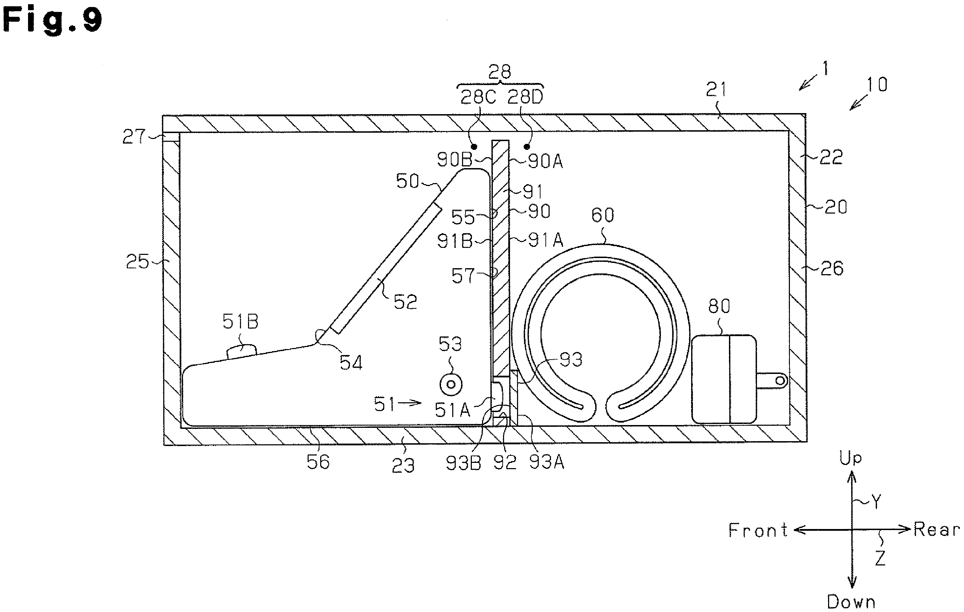

In the embodiment described above, the power button 51A is arranged on the front surface 54 of the sphygmomanometer 50, and the protector 30 is inclined with respect to the bottom wall 23 of the accommodation case 10. However, the accommodation case 10 and the sphygmomanometer body 50 may be modified as shown in Fig. 9. In the modification of Fig. 9, instead of the protector 30 of Fig. 1, the blood pressure measurement device 1 includes a protector 90 arranged parallel to the front wall 25 and the rear wall 26. The power button 51 A of the sphygmomanometer body 50 is arranged at the lower part of the rear surface 55.

The protector 90 includes a plate-like rectangular partition member 91, a plate-like rigid member 93 having higher rigidity than the partition member 91, and joining members (not shown) that join the partition member 91 and the container 22. The partition member 91, the rigid member 93, and the joining members may be formed from the same material as the partition member 31, the rigid member 33, and the joining members of the embodiment described above.

The rigid member 93 is attached to a lower part of a rear surface 91A of the partition member 91. The rear surface 91A of the partition member 91 and a front surface 93B of the rigid member 93 are fixed to each other.

In the partition member 91, an opening 92, which is a hole, is formed at the portion corresponding to the power button 51A of the sphygmomanometer body 50. The opening 92 is formed to surround the power button 51A of the sphygmomanometer body 50. The opening 92 has a greater depth than the projected amount P of the power button 51 A of the sphygmomanometer body 50. The rear surface 91A and front surface 91 B of the partition member 91 have a larger area than the rear surface 55 of the sphygmomanometer body 50.

The rear surface 90A of the protector 90 is formed by a back surface 93A of the rigid member 93 and the rear surface 91A of the partition member 91. The front surface 90B of the protector 90 is formed by the front surface 91B of the partition member 91. The rear surface 90A serves as a cuff support surface.

The protector 90 partitions the accommodation compartment 28 into two, namely, a front compartment 28C and a rear compartment 28D. The front compartment 28C, which accommodates the sphygmomanometer body 50, is surrounded by the front surface 90B of the protector 90, the lid 21, the two side walls 24, and the front wall 25 when the lid 21 is closed. The rear compartment 28D, which accommodates the cuff 60, the tube 70, and the power supply adapter 80, is surrounded by the rear surface 90A of the protector 90, the lid 21, the two side walls 24, and the rear wall 26 when the lid 21 is closed.

Each component of the electronic sphygmomanometer 40 is accommodated in the accommodation case 10 in the following manner.

The sphygmomanometer body 50 is arranged in the front compartment 28C so that the front surface 54 faces the front wall 25 of the container 22. The power button 51A is accommodated in the opening 92 of the partition member 91. The cuff 60 is arranged in the rear compartment 28D in contact with the rear surface 91A of the partition member 91. The power supply adapter 80 is arranged in the rear compartment 28D between the cuff 60 and the rear wall 26 of the container 22.

In the embodiment described above, the protector 30 is attached to the container 22 by the joining members 34, and the lower edge 31Y of the partition member 31 is fixed to the bottom wall 23 of the container 22. However, the protector 30 may be attached in a removable manner to the container 22.

The present examples and embodiments are to be considered as illustrative and not restrictive, and the invention is not to be limited to the details given herein, but may be modified within the scope and equivalence of the appended claims.

## Claims

1. A sphygmomanometer accommodation case (10) comprising:
a case body (20) that accommodates an electronic sphygmomanometer (40) including a sphygmomanometer body (50), which is provided with an operation unit (51), and a cuff (60), which is separated from the sphygmomanometer body (50); and
a partition member (31) arranged between the sphygmomanometer body (50) and the cuff (60) in the case body (20), wherein the partition member (31) includes an opening (32) arranged at a portion corresponding to the operation unit (51).

2. The sphygmomanometer accommodation case (10) according to claim 1, wherein the opening (32) is a hole formed in the partition member (31).

3. The sphygmomanometer accommodation case (10) according to claim 1 or 2,
wherein
the case body (20) includes a rectangular cuboidal-shaped container (22),
the partition member (31) is plate-like and includes a lower surface facing a bottom wall of the container (22) and inclined with respect to the bottom wall, and
the partition member (31) includes a lower edge adjacent to the bottom wall of the container (22) and an upper edge adjacent to an open top of the container (22).

4. The sphygmomanometer accommodation case (10) according to claim 3, wherein the lower edge is located in a front part of the container (22), and the upper edge is located at a rear part of the container (22).

5. The sphygmomanometer accommodation case (10) according to claim 3 or 4,
wherein a clearance is formed between the upper edge of the partition member (31) and one inner surface of the container (22).

6. The sphygmomanometer accommodation case (10) according to claim 5, wherein the clearance has a smaller width than the cuff (60).

7. The sphygmomanometer accommodation case (10) according to any one of claims 3 to 6, wherein the upper edge of the partition member (31) includes two ends, each attached to the container (22) by an elastic joining member.

8. The sphygmomanometer accommodation case (10) according to any one of claim 1 to 7, wherein
the operation unit (51) is projected from a surface of the sphygmomanometer body (50) by a projected amount, and
the opening (32) has a depth that is greater than the projected amount of the operation unit (51).

9. The sphygmomanometer accommodation case (10) according to any one of claims 1 to 8, wherein the partition member (31) includes a cuff support surface that supports the cuff (60), and the cuff support surface is defined on a rigid member having a higher rigidity than the partition member (31).

10. The sphygmomanometer accommodation case (10) according to any one of claims 1 to 9, wherein
the sphygmomanometer body (50) includes a display, and
the partition member (31) includes a transparent portion corresponding to at least the display.

11. The sphygmomanometer accommodation case (10) according to any one of claims 1 to 9, wherein
the sphygmomanometer body (50) includes a display, and
the partition member (31) covers the entire display.

12. The sphygmomanometer accommodation case (10) according to claim 1, wherein the partition member (31) partitions an interior of the case body (20) into lower and upper compartments (28B, 28A) that accommodate the sphygmomanometer body (50) and the cuff (60), respectively.

13. The sphygmomanometer accommodation case (10) according to claims 12, wherein the operation unit (51) includes a power button, and
the power button automatically enters the opening (32) of the partition member (31) when the sphygmomanometer body (50) is placed in the lower compartment (28B) of the case body (20).

14. A blood pressure measurement device comprising:
the sphygmomanometer accommodation case (10) according to any one of claims 1 to 13,
and
the electronic sphygmomanometer (40) accommodated in the accommodation case (10).

15. The blood pressure measurement device according to claim 14, wherein
the case body (20) includes a rectangular cuboidal-shaped container (22) including an accommodation compartment (28);
the partition member (31) is a plate-like member including a lower surface facing a bottom wall of the container (22) and inclined with respect to the bottom wall,
the partition member (31) includes a lower edge adjacent to the bottom wall of the container (22) and an upper edge adjacent to an open top of the container (22),
the lower edge is located in a front part of the container (22), and the upper edge is located in a rear part of the container (22),
the electronic sphygmomanometer (40) includes a bottom surface, a front surface inclined with respect to the bottom surface, and a display arranged on the inclined front surface, and
the partition member (31) partitions the accommodation compartment (28) into an upper compartment (28A), which accommodates the cuff (60), and a lower compartment (28B), which accommodates the sphygmomanometer body (50).
